# EUROPÄISCHE PATENTANMELDUNG

(11) **EP 2 801 564 A1**
(43) Veröffentlichungstag der Anmeldung: **12.11.2014**
(21) Anmeldenummer: 13166653.9
(22) Anmeldetag: 06.05.2013
(51) Int. Cl.: C07C 37/50, C07C 39/07

(54) **Decarboxylirung von 6-Methylsalicylsäure**

(71) Anmelder: LANXESS Deutschland GmbH, 50569 Köln (DE)
(72) Erfinder: Schlummer, Björn, Dr., 53225 Bonn (DE); Vogl, Nadine, Dr., 50733 Köln (DE); Dreisbach, Claus, Dr., 42799 Leichlingen (DE); Larcher, Christoph, Dr., 40476 Düsseldorf (DE)

(57) **Zusammenfassung**

Die vorliegende Erfindung betrifft ein besonders einfaches Verfahren zur Decarboxylierung von 6-Methylsalicylsäure mittels Säure- Katalysatoren, wobei als Katalysator wenigstens eine Säure aus der Gruppe der wässrigen Säuren oder der festen Säuren einsetzt, wobei als wässrige Säure H2 S04 eingesetzt wird und feste Säuren Hydroxide oder Oxide von Elementen der Gruppe 111 oder IV des Periodensystems sind.

## Beschreibung

Die vorliegende Erfindung betrifft ein besonders einfaches Verfahren zur Decarboxylierung von 6-Methylsalicylsäure (6-MSA).

Aus Berichte der Deutschen Chemischen Gesellschaft, Berlin, Verlag Chemie, Jahrg. 1883, Seite 1963 ist bekannt, 6-Methylsalicylsäure, auch als ß-Metahomosalicylsäure bezeichnet, durch Erhitzen mit konzentrierter Salzsäure auf 200°C in Kohlensäure und meta-Kresol zu spalten.

Aus WO 2012/178110 A2 ist die Decarboxylierung von 6-MSA durch Erhitzen mit Metallkatalysatoren bekannt. Explizit werden dort 20 g 6-MSA mit 2,5 g Zinkpulver decarboxyliert und 11,35 g m-Kresol in einer Reinheit von über 99,9 % erhalten.

Ausgehend von diesem Stand der Technik bestand die Aufgabe der vorliegenden Erfindung darin, alternative Katalysatoren für die Decarboxylierung von 6-MSA bereit zu stellen. Für industrielle Anwendungen wäre der Aufwand des Einsatzes von 0,0382 Mol Zink für 0,1315 Mol 6-MSA gemäß Beispiel 7 der WO 2012/178110 A2, um daraus 0,1 Mol m-Kresol zu erhalten, nicht rentabel und zudem mit hohem Aufwand hinsichtlich der Entsorgung des verbrauchten Zink-Katalysators verbunden. Für jeden Einsatz müsste neuer Zink-Katalysator bereit gestellt werden und dieser nach dem Einsatz entsorgt werden, wodurch allenfalls eine Batch-Fahrweise möglich wäre.

Es wurde nun überraschend gefunden, dass als Katalysatoren alternative Säuren zur Salzsäure (HCl) in deutlich effektiverer Weise 6-MSA decarboxylieren.

Gegenstand der vorliegenden Erfindung ist ein Verfahren zur Decarboxylierung von 6-MSA, dadurch gekennzeichnet, dass man als Katalysator wenigstens eine Säure aus der Gruppe der wässrigen Säuren oder der festen Säuren einsetzt, wobei als wässrige Säure H₂SO₄ eingesetzt wird und feste Säuren Hydroxide oder Oxide von Elementen der Gruppe III oder IV des Periodensystems sind.

Erfindungsgemäß bevorzugt entsteht meta-Kresol (m-Kresol).

Zur Klarstellung sei angemerkt, dass vom Rahmen dieser Erfindung alle nachfolgend aufgeführten, allgemeinen oder in Vorzugsbereichen genannten Definitionen und Parameter in beliebigen Kombinationen umfasst sind.

Gegenüber dem Stand der Technik haben die erfindungsgemäß einzusetzenden Katalysatoren den Vorteil, dass die Decarboxylierung vollständiger abläuft und bevorzugt m-Kresol in deutlich höherer Ausbeute erhalten wird sowie ein Eintrag von Schwermetallen ins Reaktionsmedium sowie ins m-Kresol unterbleibt. Die Anwesenheit von Schwermetallen beispielsweise im m-Kresol kann dessen Einsatz in der Pharmazie oder im Pflanzenschutz erheblich einschränken, wenn nicht das m-Kresol umfangreichen Aufarbeitungsschritten zuvor unterzogen wird.

In einer Ausführungsform betrifft die vorliegende Erfindung ein Verfahren zur Herstellung von m-Kresol indem man ausgehend von 6-MSA wenigstens eine Säure aus der Gruppe der wässrigen Säuren oder der festen Säuren einsetzt, wobei als wässrige Säure H₂SO₄ eingesetzt wird.

Aus DE69209751T2 ist bekannt, dass sogenannte "feste Säuren", insbesondere Siliciumdioxid, Aluminiumoxid, Zeolithe, Mordenit oder Siliciumdioxid-Aluminiumoxid als heterogene Katalysatoren für die Isomerisierung von linearen Paraffinen zu verzweigten Paraffinen verwendet werden können. Der erfindungsgemäß verwendete Katalysator ist ein festes, stark saures Material.

Aus WO 2005/099896 A1 sind Aluminiumoxid, Zinnoxid, Titandioxid, Zirkoniumdioxid, Molybdänoxid, Wolframoxid als "feste Säuren" bekannt.

Erfindungsgemäß sind feste Säuren Hydroxide oder Oxide von Elementen der Gruppe III oder IV des Periodensystems, bevorzugt Hydroxide oder Oxide von Titan, Zirconium, Silicium, Germanium, Zinn, Aluminium, Gallium oder Indium, besonders bevorzugt Oxide von Titan, Zirconium, Silicium, Germanium, Zinn, Aluminium, Gallium oder Indium, ganz besonders bevorzugt Oxide von Titan, Zirconium, Silicium, Zinn oder Aluminium.

Das erfindungsgemäße Verfahren erlaubt aber auch den Einsatz von Supersäurekatalysatoren als "feste Säuren". Bevorzugte Supersäuren für den erfindungsgemäßen Einsatz sind feste Säuren, von denen bekannt ist, dass ihre Azidität stärker als die von 100 %iger H₂SO₄ ist, d.h. H₀ < -12. Erfindungsgemäß bevorzugt zu verwendende feste Supersäuren sind sulfatiertes Zirconiumoxid, sulfatiertes Titanoxid, sulfatiertes Eisenoxid oder halogeniertes Aluminiumoxid, insbesondere fluoriertes Al₂O₃. Weitere Typen von festen Supersäuren sind starke Lewissäuren wie SbF₅, SbCl₅, SbF₅/HF auf einem festen Trägermaterial wie Siliciumdioxid, Aluminiumoxid oder Zirconiumoxid oder Kombinationen davon.

Die vorliegende Erfindung betrifft ferner die Verwendung von wenigstens einer Säure aus der Gruppe der wässrigen Säuren oder der festen Säuren als Katalysator zur Decarboxylierung von 6-MSA, wobei als wässrige Säure H₂SO₄ eingesetzt wird und feste Säuren Hydroxide oder Oxide von Elementen der Gruppe III oder IV des Periodensystems sind.

In einer Ausführungsform betrifft die vorliegende Erfindung ein Verfahren zur Decarboxylierung von 6-MSA, dadurch gekennzeichnet, dass man als Katalysator wenigstens eine feste Säure einsetzt und feste Säuren die Hydroxide oder Oxide von Elementen der Gruppe III oder IV des Periodensystems sind, bevorzugt Hydroxide oder Oxide von Titan, Zirconium, Silicium, Germanium, Zinn, Aluminium, Gallium oder Indium, besonders bevorzugt Oxide von Titan, Zirconium, Silicium, Germanium, Zinn, Aluminium, Gallium oder Indium, ganz besonders bevorzugt Oxide von Titan, Zirconium, Silicium, Zinn oder Aluminium.

In einer Ausführung der vorliegenden Erfindung wird als Katalysator H₂SO₄ eingesetzt.

In einer Ausführungsform wird das erfindungsgemäße Verfahren lösungsmittelfrei durchgeführt und ist damit den Verfahren des Standes der Technik in der Durchführung technisch überlegen.

In einer Ausführungsform wird das erfindungsgemäße Verfahren bei einer Temperatur im Bereich oberhalb der Schmelztemperatur der 6-MSA von 1780°C-220°C, bevorzugt im Bereich von 175°C-210°C durchgeführt. Die experimentellen Untersuchungen zur vorliegenden Erfindung wurden bei 195°C durchgeführt.

In einer Ausführungsform kann 6-MSA auf petrochemischer Basis oder aus mikrobiologischen Syntheseprozessen eingesetzt werden.

In einer Ausführungsform kann das erfindungsgemäße Verfahren in Gegenwart der 0,5-bis 15-fachen Gewichtsmenge Wasser, bezogen auf die einzusetzende Menge 6-MSA durchgeführt werden. Bevorzugt beträgt diese Menge das 0,8- bis 6-fache.

In einer Ausführungsform kann das erfindungsgemäße Verfahren in Gegenwart der bis zu 15-fachen Gewichtsmenge einer oder mehrerer zusätzlicher Säuren bezogen auf die eingesetzte Menge 6-MSA durchgeführt werden. Vorzugsweise beträgt diese Menge das 0,01- bis 5-fache.

Üblicherweise erhitzt man die 6-MSA gegebenenfalls in Gegenwart von Wasser und in Gegenwart von wenigstens einem der erfindungsgemäß einzusetzenden Säure-Katalysatoren, vorzugsweise auf eine Temperatur von 170 bis 220 °C. Wenn die Reaktionstemperatur bei Normaldruck über der Siedetemperatur des Reaktionsgemisches liegt, ist es erforderlich, die Decarboxylierung in einem druckfesten Reaktor, bevorzugt in einem Autoklaven, durchzuführen.

Die Reaktionszeiten können in einem weiten Bereich variiert werden und vorzugsweise im Bereich von 1 bis 60 Stunden liegen. Im Allgemeinen kann bei höheren Reaktionstemperaturen mit kürzeren Reaktionszeiten gearbeitet werden als bei tieferen Reaktionszeiten. Es ist ein wesentliches Merkmal des erfindungsgemäßen Verfahrens, dass man es ohne Zusätze von organischen Lösungsmitteln durchführt.

Das nach der Durchführung der erfindungsgemäßen Decarboxylierung vorliegende Reaktionsmedium wird in einer bevorzugten Ausführungsform direkt destilliert, wodurch man bevorzugt m-Kresol isoliert. Man erhält so im Allgemeinen über 97 % reines m-Kresol. Der Siedepunkt von m-Kresol liegt bei 203°C, so dass entsprechend hohe Temperaturen für die Destillation des Reaktionsmediums gewählt werden müssen.

Im Hinblick auf den eingangs geschilderten Stand der Technik ist es beim erfindungsgemäßen Verfahren ausgesprochen überraschend, dass es mit den erfindungsgemäßen Säuren als Katalysatoren und ohne organische Lösungsmittel gelingt, mit guten Ausbeuten aus 6-MSA selektiv die Carboxylgruppe abzuspalten und das bevorzugte Decarboxylierungsprodukt m-Kresol in hohen Ausbeuten und hohen Reinheiten zu erhalten.

Das bevorzugte Reaktionsprodukt m-Kresol ist ein wertvolles Zwischenprodukte zur Herstellung von Pflanzenschutzwirkstoffen und Pharmawirkstoffen. m-Kresol findet als Fungizid in der Landwirtschaft Anwendung. Kresole werden auch verwendet, um daraus Kunst- und Farbstoffe, Kunstharze (Kresolharze) und Arzneimittel herzustellen.

### Beispiele

### Beispiel 1

### Allgemeine Arbeitsvorschrift:

1,0 g (6,6 mmol) 6-Methylsalicylsäure wurden mit der angegebenen Menge des zu verwendenden Katalysators innig vermengt und dann ohne Zugabe von Lösungsmittel unter inerter Atmosphäre auf 195 °C erhitzt. Das Gemisch wurde 6 h bei dieser Temperatur gehalten und dann auf Raumtemperatur abgekühlt. Das erhaltene Produktgemisch wurde mit 50 g Toluol extrahiert und das erhaltene Produktgemisch nach Silylierung mittels Gaschromatopgraphie untersucht und die Produktverhältnisse bestimmt.

In der Tabelle 1 bedeutet 5g Versuch, dass in obiger Versuchsvorschrift 5g 6-MSA mit 100 mol-% Katalysator umgesetzt wurden.

Für die Ermittlung der Ausbeute wurde die Vorschrift im fünffachen Maßstab durchgeführt und die erhaltene organische Produktphase vollständig vom Lösungsmittel befreit, um die Produktmenge per Auswaage zu bestimmen.

Das Produkt wurde dann nach Silylierung mittels Gaschromatographie untersucht. Die Ergebnisse der verschiedenen Umsätze sind in Tabelle 1 dargestellt. Jeweils angegeben ist der Umsatz von 6-MSA zu m-Kresol in Prozent.

**Tabelle 1**

| **Eingesetzte Menge des Katalysators** | | | | |
|---|---|---|---|---|
| **Katalysator** | 100 mol-% | 50 mol-% | 10 mol-% | 5g Versuch |

| **Säuren** | | | | |
|---|---|---|---|---|
| H₂SO₄ | 100 | 100 | 100 | 29 |
| HCl (37%) - Stand der Technik | 41 | | | |
| Kein Katalysator | 34 | | | 12 |

100 mol-% bedeuten, dass relativ zu der eingesetzten Molmenge 6-Methylsalicylsäure die gleiche Molmenge (100 mol-%) des Katalysators eingesetzt wurden. 50 mol-% bedeuten, dass relativ zu der eingesetzten Molmenge 6-Methylsalicylsäure die halbe molare Menge (50 mol-%) des Katalysators eingesetzt wurden. 10 mol-% bedeuten, dass relativ zu der eingesetzten Molmenge 6-Methylsalicylsäure ein Zehntel der molaren Menge (10 mol-%) des Katalysators eingesetzt wurden.

Die in Tabelle 1 aufgelisteten Säure-Katalysatoren sind für den Fachmann gängige und leicht zugängliche Edukte.

Die mit den erfindungsgemäßen Katalysatoren, insbesondere der wässrigen Säure H₂SO₄, erzielten Ergebnisse zeigen überraschenderweise eine gegenüber dem Stand der Technik deutlich höhere Ausbeute an m-Kresol.

### Beispiel 2

### Allgemeine Arbeitsvorschrift:

1,0 g (6,6 mmol) 6-Methylsalicylsäure wurden mit der 0,5 g des zu verwendenden Katalysators innig vermengt und dann ohne Zugabe von Lösungsmittel unter inerter Atmosphäre auf 195 °C erhitzt. Das Gemisch wurde 6 h bei dieser Temperatur gehalten und dann auf Raumtemperatur abgekühlt. Das erhaltene Produktgemisch wurde mit 50 g Toluol extrahiert und das erhaltene Produktgemisch nach Silylierung mittels Gaschromatopgraphie untersucht und die Produktverhältnisse bestimmt. Jeweils angegeben ist der Umsatz von 6-MSA zu m-Kresol in Prozent.

**Tabelle 2**

| **Eingesetzte Menge des Katalysators** | |
|---|---|
| **Katalysator** | 0,5 g Katalysator |
| Feste Säure | |
| Sulfatiertes ZrO₂ | |
| Norpro SZ61192 | 100 |
| Sasol Siralox 10 | 100 |
| Wolfram dotiertes ZrO₂ | |
| Norpro SZ61143 | 100 |
| Al₂O₃ BASF D10-10 | 100 |
| HCl (37%) - Stand der Technik | 41 |
| Kein Katalysator | 34 |

Die in Tabelle 2 aufgelisteten Feste-Säure-Katalysatoren sind für den Fachmann gängige und leicht zugängliche Edukte.

Die mit den erfindungsgemäßen Katalysatoren erzielten Ergebnisse zeigen überraschenderweise eine gegenüber dem Stand der Technik deutlich höhere Ausbeute an m-Kresol.

### Beispiel 3

### Allgemeine Arbeitsvorschrift:

1,0 g (6,6 mmol) 6-Methylsalicylsäure wurden mit der 0,5 g des zu verwendenden Katalysators innig vermengt und dann ohne Zugabe von Lösungsmittel unter inerter Atmosphäre auf 175 °C erhitzt. Das Gemisch wurde 6 h bei dieser Temperatur gehalten und dann auf Raumtemperatur abgekühlt. Das erhaltene Produktgemisch wurde mit 50 g Toluol extrahiert und das erhaltene Produktgemisch nach Silylierung mittels Gaschromatopgraphie untersucht und die Produktverhältnisse bestimmt. Jeweils angegeben ist der Umsatz von 6-MSA zu m-Kresol in Prozent.

**Tabelle 3**

| **Eingesetzte Menge des Katalysators** | |
|---|---|
| **Katalysator** | 0,5 g Katalysator |
| Feste Säure | |
| Sulfatiertes ZrO₂ | |
| Norpro SZ61192 | 83,9 |
| Sasol Siralox 10 | 100 |
| Wolfram dotiertes ZrO₂ | |
| Norpro SZ61143 | 100 |
| Al₂O₃ | |
| BASF D10-10 | 100 |

### Beispiel 4

### Allgemeine Arbeitsvorschrift:

5,0 g (33 mmol) 6-Methylsalicylsäure wurden mit der 2,5 g des zu verwendenden Katalysators innig vermengt und dann ohne Zugabe von Lösungsmittel unter inerter Atmosphäre auf 195 °C erhitzt. Das Gemisch wurde 6 h bei dieser Temperatur gehalten und dann auf Raumtemperatur abgekühlt. Das erhaltene Produktgemisch wurde mit 250 g Toluol extrahiert und das erhaltene Produktgemisch nach Silylierung mittels Gaschromatopgraphie untersucht und die Produktverhältnisse bestimmt. Jeweils angegeben ist der Umsatz von 6-MSA zu m-Kresol in Prozent. Die Suspension wurde filtriert und das Filtrat anschließend vom Toluol befreit. Der eingedampfte Rückstand wurde in 25 g THF (Tetrahydrofuran) aufgenommen und per quantitativer HPLC analysiert

**Tabelle 4**

| **Eingesetzte Menge des Katalysators** | | | | |
|---|---|---|---|---|
| **Katalysator** | 2,,5 g Katalysator | | | |
| Feste Säure | Umsatz [%] | Auswaage [g] | Gehalt nach HPLC [%] | % der Theorie |
| Sasol Siralox 10 | 100 | 3,56 | 39 | 39 |
| Wolfram dotiertes ZrO₂ Norpro SZ61143 | 100 | 5,97 | 34 | 57 |

## Patentansprüche

1. Verfahren zur Decarboxylierung von 6-MSA, **dadurch gekennzeichnet, dass** man als Katalysator wenigstens eine Säure aus der Gruppe der wässrigen Säuren oder der festen Säuren einsetzt, wobei als wässrige Säure H₂SO₄ eingesetzt wird und feste Säuren Hydroxide oder Oxide von Elementen der Gruppe III oder IV des Periodensystems sind.

2. Verfahren gemäß Anspruch 1, **dadurch gekennzeichnet, dass** als feste Säuren Hydroxide oder Oxide von Titan, Zirconium, Silicium, Germanium, Zinn, Aluminium, Gallium oder Indium, bevorzugt Oxide von Titan, Zirconium, Silicium, Germanium, Zinn, Aluminium, Gallium oder Indium, besonders bevorzugt Oxide von Titan, Zirconium, Silicium, Zinn oder Aluminium eingesetzt werden.

3. Verfahren gemäß Anspruch 2, **dadurch gekennzeichnet, dass** man feste Säuren Säuren einsetzt, deren Azidität stärker als die von 100 %iger H₂SO₄ ist, d.h. Ho < -12.

4. Verfahren gemäß Anspruch 3, **dadurch gekennzeichnet dass** sulfatiertes Zirconiumoxid, sulfatiertes Titanoxid, sulfatiertes Eisenoxid oder halogeniertes Aluminiumoxid, insbesondere fluoriertes Al₂O₃ eingesetzt werden.

5. Verfahren gemäß Anspruch 1, **dadurch gekennzeichnet, dass** man als Katalysator H₂SO₄ einsetzt.

6. Verfahren gemäß der Ansprüche 1 bis 5, **dadurch gekennzeichnet, dass** man dieses bei einer Temperatur im Bereich von 180°C-200°C, bevorzugt im Bereich von 190°C-200°C durchgeführt.

7. Verfahren gemäß der Ansprüche 1 bis 6, **dadurch gekennzeichnet, dass** man dieses in Gegenwart der 0,5- bis 15-fachen Gewichtsmenge Wasser, bezogen auf die einzusetzende Menge 6-MSA durchführt, bevorzugt beträgt diese Menge das 0,8- bis 6-fache.

8. Verfahren gemäß der Ansprüche 1 bis 7, **dadurch gekennzeichnet, dass** man dieses in Gegenwart zusätzlicher Metallkatalysatoren durchführt, bevorzugt in Gegenwart von Metallkatalysatoren auf Basis der Metalle der Reihe Zn, Fe, Cu, Ni, Co, Pd, Mo, Ru oder deren chemische Verbindungen mit Nichtmetallen bzw. Salze der Reihe Oxide, Chloride, Acetate oder deren Komplexe mit CO oder Dibenzylidenaceton.

9. Verfahren gemäß der Ansprüche 1 bis 8, **dadurch gekennzeichnet, dass** das Reaktionsprodukt m-Kresol ist.

10. Verfahren gemäß Anspruch 9, **dadurch gekennzeichnet, dass** man das nach der Decarboxylierung vorliegende Reaktionsmedium direkt destilliert und das m-Kresol isoliert.

11. Verwendung von wenigstens einer Säure aus der Gruppe der wässrigen Säuren oder der festen Säuren als Katalysator zur Decarboxylierung von 6-MSA, wobei als wässrige Säure H₂SO₄ eingesetzt wird und feste Säuren Hydroxide oder Oxide von Elementen der Gruppe III oder IV des Periodensystems sind.

12. Verwendung nach Anspruch 11, **dadurch gekennzeichnet, dass** als feste Säuren Hydroxide oder Oxide von Titan, Zirconium, Silicium, Germanium, Zinn, Aluminium, Gallium oder Indium, bevorzugt Oxide von Titan, Zirconium, Silicium, Germanium, Zinn, Aluminium, Gallium oder Indium, besonders bevorzugt Oxide von Titan, Zirconium, Silicium, Zinn oder Aluminium eingesetzt werden.
